(19) Europäisches Patentamt
European Patent Office
Office européen des brevets

(11) **EP 1 110 537 A2**

(12) **DEMANDE DE BREVET EUROPEEN**

(43) Date de publication:
**27.06.2001 Bulletin 2001/26**

(51) Int Cl.7: **A61K 7/48**

(21) Numéro de dépôt: **00403320.5**

(22) Date de dépôt: **28.11.2000**

(84) Etats contractants désignés:
**AT BE CH CY DE DK ES FI FR GB GR IE IT LI LU MC NL PT SE TR**
Etats d'extension désignés:
**AL LT LV MK RO SI**

(30) Priorité: **20.12.1999 FR 9916074**

(71) Demandeur: **L'OREAL**
**75008 Paris (FR)**

(72) Inventeurs:
- **Chevalier, Véronique**
  **94440 Villecresnes (FR)**
- **Pham, Dang-Man**
  **94370 Sucy-en-Brie (FR)**

(74) Mandataire: **Renard, Emmanuelle**
**L'OREAL-DPI**
**6 rue Bertrand Sincholle**
**92585 Clichy Cedex (FR)**

(54) **Composition cosmétique comprenant le N-éthyloxycarbonyl-4-amino-phénol et l'arbutine ou ses dérivés et/ou l'acide ellagique ou ses dérivés**

(57) L'invention concerne une composition comprenant, dans un milieu physiologiquement acceptable, le N-éthyloxycarbonyl-4-para-aminophénol et au moins un agent dépigmentant choisi parmi : l'arbutine et ses dérivés et/ou l'acide ellagique et ses dérivés.

Ces associations d'actifs présentent une synergie dans la dépigmentation ou le blanchiment de la peau, des poils et/ou des cheveux.

La composition selon l'invention renferme en outre avantageusement au moins un filtre UV et/ou au moins un agent desquamant.

EP 1 110 537 A2

## Description

La présente invention concerne une composition comprenant, dans un milieu physiologiquement acceptable, le N-éthyloxycarbonyl-4-para-aminophénol et au moins un agent dépigmentant choisi parmi : l'arbutine et ses dérivés et/ou l'acide ellagique et ses dérivés.

Depuis plusieurs années, des efforts importants ont été déployés en vue de proposer des substances dépigmentantes topiques inoffensives présentant une bonne efficacité.

Ces substances sont particulièrement recherchées en vue de traiter les hyperpigmentations régionales par hyperactivité mélanocytaire telles que les mélasmas idiopathiques, survenant lors de la grossesse ("masque de grossesse" ou chloasma) ou d'une contraception oestro-progestative, les hyperpigmentations localisées par hyperactivité et prolifération mélanocytaire bénigne, telles que les taches pigmentaires séniles dites lentigo actiniques, les hyperpigmentations ou dépigmentations accidentelles, éventuellement dues à la photosensibilisation ou à la cicatrisation post-lésionnelle, ainsi que certaines leucodermies, telles que le vitiligo. Pour ces dernières (les cicatrisations pouvant aboutir à une cicatrice donnant à la peau un aspect plus blanc et les leucodermies), à défaut de pouvoir repigmenter la peau lésée, on achève de dépigmenter les zones de peau normale résiduelle pour donner à l'ensemble de la peau une teinte blanche homogène.

Divers agents dépigmentants ont ainsi été proposés dans l'art antérieur.

En particulier, il a été démontré par la Demanderesse que certains dérivés d'aminophénol avaient la propriété d'inhiber la mélanogénèse même à faibles concentrations, sans faire preuve de cytotoxicité. Ces composés, ainsi que leur procédé de préparation, sont décrits dans la demande de brevet WO 99/10318.

D'autres dépigmentants bien connus sont constitués par l'hydroquinone et ses dérivés tels que l'arbutine et ses esters ; l'acide ascorbique et ses dérivés, notamment glycosylés ; l'acide kojique et ses esters ; l'acide ellagique et ses dérivés ; les extraits de plantes, et notamment de réglisse, de mûrier ou de scutellaire ; ainsi que le glutathion, la cystéine et leurs précurseurs.

La demanderesse a maintenant découvert de manière inattendue que l'association de l'un des dérivés d'aminophénol précités, à savoir le N-éthyloxycarbonyl-4-para-aminophénol, avec l'arbutine ou un de ses dérivés et/ou l'acide ellagique ou un de ses dérivés présentait un pouvoir dépigmentant supérieur à celui de ces composés, considérés séparément. En d'autres termes, cette association potentialise l'efficacité de ces composés qui produisent un effet de synergie dans le blanchiment ou la dépigmentation de la peau, des poils ou des cheveux.

La présente invention a donc pour objet une composition comprenant, dans un milieu physiologiquement acceptable, le N-éthyloxycarbonyl-4-para-aminophénol en combinaison avec au moins un agent dépigmentant choisi parmi l'arbutine et ses dérivés et/ou l'acide ellagique et ses dérivés.

Par "milieu physiologiquement acceptable", on entend un milieu convenant à une application topique sur la peau ou ses phanères, c'est-à-dire compatible avec la peau, les poils, les cheveux, les ongles et les muqueuses.

L'arbutine est un glucoside d'hydroquinone ayant pour formule :

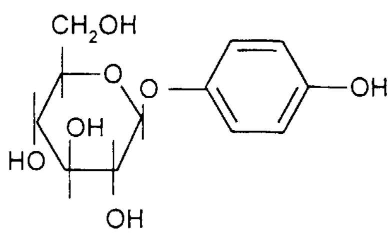

Par "dérivé d'arbutine", on entend de préférence les esters d'arbutine tels que les mono-esters d'arbutine décrits dans les demandes EP-0 597 776 et EP-0 895 779 qui sont incorporées ici par référence, et les esters d'arbutoside décrits dans la demande EP-0 524 109, dont le contenu est également incorporé ici par référence.

L'acide ellagique et ses dérivés comprennent les composés de formule (I) suivante :

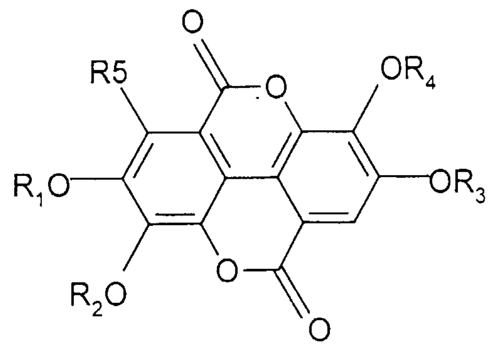

(I)

où :

$R_1$ à $R_4$ sont des atomes d'hydrogène, un groupe alkyle ayant de 1 à 20 atomes de carbone, un groupe alcoxy ayant de 1 à 20 atomes de carbone, un reste de poly(oxyde d'éthylène) ou de poly(oxyde de propylène) ou un reste de sucre, et
$R_5$ est un atome d'hydrogène, un groupe hydroxyle ou un groupe alcoxy ayant de 1 à 8 atomes de carbone.

Ces composés sont décrits dans le brevet US-5,073,545, et leur mode de préparation est divulgué dans la demande JP-53-14605.

L'invention a également pour objet l'utilisation de cette composition dans une préparation cosmétique dépigmentante et/ou blanchissante de la peau humaine, des poils ou des cheveux.

Elle a en outre pour l'objet l'utilisation de cette composition pour la fabrication d'une préparation dépigmentante et/ou blanchissante de la peau humaine, des poils ou des cheveux.

La présente invention se rapporte également à un procédé cosmétique de dépigmentation et/ou de blanchiment de la peau humaine, des poils ou des cheveux consistant à appliquer sur la peau, les poils ou les cheveux une composition selon l'invention.

Le N-éthyloxycarbonyl-4-para-aminophénol et l'acide ellagique (ou son dérivé) et/ou l'arbutine (ou son dérivé) sont présents, dans la composition selon l'invention, en une quantité telle qu'ils agissent en synergie pour conférer à la composition un effet dépigmentant supérieur à celui obtenu avec une composition ne renfermant que l'un de ces composés. Par exemple, le N-éthyloxycarbonyl-4-para-aminophénol peut être présent en une quantité allant de 0,01 à 10% en poids, et de préférence de 0,5 à 3% en poids, par rapport au poids total de la composition. De son côté, l'arbutine ou son dérivé peut représenter de 0,01 à 5% en poids, préférentiellement de 0,1 à 2% en poids, par rapport au poids total de la composition. Enfin, l'acide ellagique ou son dérivé peut représenter de 0,001 à 10% en poids, et de préférence de 0,005 à 3% en poids, par rapport au poids total de la composition.

La composition de l'invention peut se présenter sous toutes les formes galéniques normalement utilisées pour une application topique, notamment sous forme d'une solution aqueuse, hydroalcoolique ou huileuse, d'une émulsion huile-dans-eau ou eau-dans-huile ou multiple, d'un gel aqueux ou huileux, d'un produit anhydre liquide, pâteux ou solide, d'une dispersion d'huile dans une phase aqueuse à l'aide de sphérules, ces sphérules pouvant être des nanoparticules polymériques telles que les nanosphères et les nanocapsules ou, mieux, des vésicules lipidiques de type ionique et/ou non-ionique.

Le dérivé d'aminophénol et l'acide ellagique mis en oeuvre dans la présente invention peuvent présenter des problèmes de solubilité dans certaines compositions cosmétiques. Pour y remédier, il peut être utile, selon une forme d'exécution préférée, de solubiliser le N-éthyloxycarbonyl-4-para-aminophénol dans un solubilisant choisi parmi un ester d'acide gras et de polyol oxyalkyléné, un alcool gras oxyalkyléné et leurs mélanges, avant de l'introduire dans la phase aqueuse ou dans la phase grasse de la composition selon l'invention. Par ailleurs, il peut être avantageux de dissoudre l'acide ellagique dans une solution basique de pH supérieur à 14, puis d'acidifier cette solution jusqu'à un pH compris entre environ 6 et 8 préalablement à son incorporation dans la composition de l'invention.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sur la peau ou sur les cheveux sous forme d'aérosol. Elle peut également se présenter sous forme solide, et par exemple sous forme de stick. Elle peut être utilisée comme produit de soin et/ou comme produit de maquillage pour la peau. Elle peut également être sous une forme de shampooing ou d'après-shampooing.

De façon connue, la composition de l'invention peut contenir également les adjuvants habituels dans le domaine cosmétique, tels que les gélifiants hydrophiles ou lipophiles, les actifs hydrophiles ou lipophiles, les conservateurs, les antioxydants, les solvants, les parfums, les charges, les filtres, les pigments, les absorbeurs d'odeur et les matières colorantes. Les quantités de ces différents adjuvants sont celles classiquement utilisées dans le domaine considéré, et par exemple de 0,01 à 20% du poids total de la composition. Ces adjuvants, selon leur nature, peuvent être introduits dans la phase grasse, dans la phase aqueuse, dans les vésicules lipidiques et/ou dans les nanoparticules. En tout état de cause, ces adjuvants, ainsi que leurs proportions, seront choisis de manière à ne pas nuire aux propriétés recherchées de l'association d'agents dépigmentants selon l'invention.

Lorsque la composition de l'invention est une émulsion, la proportion de la phase grasse peut aller de 5 à 80 % en poids, et de préférence de 5 à 50 % en poids par rapport au poids total de la composition. Les huiles, les émulsionnants et les coémulsionnants utilisés dans la composition sous forme d'émulsion sont choisis parmi ceux classiquement utilisés dans le domaine considéré. L'émulsionnant et le coémulsionnant sont présents, dans la composition, en une proportion allant de 0,3 à 30 % en poids, et de préférence de 0,5 à 20 % en poids par rapport au poids total de la composition.

Comme huiles utilisables dans l'invention, on peut citer les huiles minérales (huile de vaseline), les huiles d'origine végétale (huile d'avocat, huile de soja), les huiles d'origine animale (lanoline), les huiles de synthèse (perhydrosqualène), les huiles siliconées (cyclométhicone) et les huiles fluorées (perfluoropolyéthers). On peut aussi utiliser comme matières grasses des alcools gras (alcool cétylique), des acides gras, des cires (cire de carnauba, ozokérite).

Comme émulsionnants et coémulsionnants utilisables dans l'invention, on peut citer par exemple les esters d'acide gras et de polyéthylène glycol tels que le stéarate de PEG-20, et les esters d'acide gras et de glycérine tels que le stéarate de glycéryle.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

Comme actifs, la composition selon l'invention comprend de préférence au moins un filtre UV et/ou au moins un agent kératolytique et/ou desquamant.

L'invention va maintenant être illustrée à l'aide des exemples non limitatifs qui suivent.

**Exemple 1 : Mise en évidence de l'effet synergique des composés selon l'invention**

Un test biologique in vitro (inhibition de l'activité de la tyrosinase de champignon) a mis en évidence l'activité dépigmentante des associations de composés selon l'invention.

Ce test consiste à mesurer l'absorbance (densité optique DO) à 475 nm en fonction du temps pour chacun des actifs dépigmentants suivants :

- N-éthyloxycarbonyl-4-para-aminophénol
- arbutine,

dissous (à différentes concentrations) dans un solvant approprié en présence d'un tampon phosphate, de 1 ml de L-tyrosine à 2,0 x $10^{-3}$ M dans un tampon phosphate, de 0,1 ml de L-Dopa à 1,0 $\times$ $10^{-4}$ M dans un tampon phosphate et de 50 µl de solution de tyrosinase de champignon à 1 g/l dans un tampon phosphate, comparés à un témoin constitué du solvant utilisé à la même concentration dans le tampon phosphate. Un pourcentage d'inhibition est ensuite déterminé par l'équation suivante :

$$\%\text{inhibition} = \frac{DO_{max,\ 475\ nm}\ (\text{témoin}) - DO_{max,\ 475\ nm}\ (\text{actif})}{DO_{max,\ 475\ nm}\ (\text{témoin})} \times 100$$

Pour chacun des trois composés actifs précédents, on détermine ensuite la concentration molaire pour laquelle est observée une valeur d'inhibition de 25%. On obtient ainsi une concentration $C_1$ de N-éthyloxycarbonyl-4-para-aminophénol et une concentration $C_2$ d'arbutine où :

$$C_1 = 1{,}25 \times 10^{-4}\ \text{M (solvant : éthanol)}$$

$$C_2 = 3{,}33 \times 10^{-4}\ \text{M (solvant : eau)}$$

De son côté, l'acide ellagique a été utilisé à sa limite supérieure de solubilité dans le solvant (DMSO), soit à

une concentration $C_3 = 1,35 \times 10^{-4}$ M. A cette concentration, l'acide ellagique n'a pas d'effet inhibiteur de l'activité de la tyrosinase.

On réalise ensuite le mélange : du N-éthyloxycarbonyl-4-para-aminophénol et de l'arbutine dans les concentrations $C_1$ et $C_2$ ; et du N-éthyloxycarbonyl-4-para-aminophénol et de l'acide ellagique dans les concentrations respectives $C_1$ et $C_3$. On calcule alors le pourcentage d'inhibition théorique du mélange (ou IC théorique), correspondant à la somme des pourcentages d'inhibition observés pour les composés individuels, et on détermine le pourcentage d'inhibition obtenu (ou IC obtenu).

Les résultats sont les suivants :

Mélange $C_1 + C_2$ :

IC théorique = 47,3 %
IC obtenu = 58,0 %

Mélange $C_1 + C_3$ :

IC théorique = 24,8 %
IC obtenu = 59,0 %

Compte tenu de la précision de ce test (de l'ordre de 5%), l'effet de synergie des deux composés en association est ainsi clairement mis en évidence.

**Exemple 2 (comparatif)**

Le test décrit dans l'Exemple 1 a été reproduit, excepté que le N-éthyloxycarbonyl-4-para-aminophénol a été remplacé par le N-cholestéryloxycarbonyl-4-para-aminophénol.

Les résultats obtenus sont les suivants :

$$C'_1 = 6,67 \times 10^{-5} \text{ M}$$

$$C_2 = 3,33 \times 10^{-4} \text{ M}$$

Mélange $C'_1 + C_2$

IC théorique = 22,5 %
IC obtenu = 23,2 %

Il est clair que, compte tenu du fait que l'erreur relative de la mesure est de 5%, on n'a pas observé d'effet synergique pour l'association de ce dérivé d'aminophénol avec l'arbutine.

**Exemple 3 (comparatif)**

On a déterminé, de la même manière que dans l'Exemple 1, l'effet dépigmentant d'un mélange de N-éthyloxycarbonyl-4-para-aminophénol et d'hydroquinone, qui est un dépigmentant bien connu dont la structure chimique est proche de celle de l'arbutine (l'arbutine étant un glucoside d'hydroquinone).

On a observé une inhibition de 25% de l'activité de la tyrosinase pour une concentration $C_4$ d'hydroquinone, où $C_4 = 8 \times 10^{-5}$ M. Le pourcentage d'inhibition théorique était de 49,2% pour le mélange des deux composés dans les concentrations $C_1$ et $C_4$. Le pourcentage d'inhibition obtenu était de 29,4%.

Aucun effet de synergie n'a ainsi été mis en évidence.

**Exemple 4 : Emulsion H/E aux oléosomes**

On prépare la composition suivante comme décrit dans la demande EP-A-705 593. Les quantités mentionnées ci-dessous sont en pourcentage pondéral.

| | |
|---|---|
| N-éthyloxycarbonyl-4-para-aminophénol | 0,5 % |
| Arbutine | 1,0 % |
| Heptanoate de stéaryle et octanoate de stéaryle | 5,5 % |
| Huiles végétales | 11,6 % |
| Conservateurs | 0,29 % |
| Filtre UVA | 1,9% |
| Triéthanolamine | 0,75 % |

(suite)

| | |
|---|---|
| EDTA disodique | 0,05 % |
| Cyclopentasiloxane | 3,7 % |
| Glycérine | 3 % |
| Tristéarate de sucrose | 2 % |
| Monostéarate de sorbitane oxyéthyléné (4 OE) | 1,35 % |
| Acide stéarique | 1 % |
| Huile de ricin hydrogénée oxyéthylénée (60 OE) | 2,5 % |
| Acétate de tocophéryle | 0,5 % |
| Eau qsp. | 100 % |

On obtient une crème blanchissante pour le visage.

**Exemple 5 : gel dépigmentant**

On prépare la composition suivante de manière classique.

| | |
|---|---|
| N-éthyloxycarbonyl-4-para-aminophénol | 0,5 % |
| Acide ellagique | 0,1 % |
| Conservateurs | 0,65 % |
| Filtre UVB | 0,5 % |
| EDTA disodique | 0,1 % |
| Hydroxyde de sodium | 0,11 % |
| Carbomers | 0,4 % |
| Cyclohexasiloxane | 5 % |
| Glycérine | 10 % |
| Ethanol | 2,5 % |
| Eau qsp. | 100 % |

On obtient un gel bien adapté à l'atténuation des lentigo actiniques sur les mains.

**Exemple 6 : émulsion H/E sans tensioactif**

On prépare la composition suivante de manière classique.

| | |
|---|---|
| N-éthyloxycarbonyl-4-para-aminophénol | 0,5 % |
| Arbutine | 2 % |
| Huiles végétales | 12 % |
| Conservateurs | 0,3 % |
| Filtre UVA | 2 % |
| Filtre UVB | 4 % |
| EDTA disodique | 0,05 % |
| Copolymère diglycol/CHDM/isophtalates/SIP (Eastman AQ 55S d'EASTMAN CHEMICAL) | 2 % |
| Cyclopentasiloxane | 6 % |
| Glycérine | 5 % |
| Ethanol | 10 % |
| Eau | qsp. 100 % |

On obtient une crème blanchissante convenant particulièrement bien aux peaux sensibles ou réactives.

**Revendications**

1. Composition comprenant, dans un milieu physiologiquement acceptable, le N-éthyloxycarbonyl-4-para-aminophénol et au moins un agent dépigmentant choisi parmi l'arbutine et ses dérivés et/ou l'acide ellagique et ses dérivés.

2. Composition selon la revendication 1, caractérisée en ce qu'elle renferme de 0,01 à 10% en poids de N-éthyloxycarbonyl-4-para-aminophénol par rapport au poids total de la composition.

3. Composition selon la revendication 2, caractérisée en ce qu'elle renferme de 0,5 à 3% en poids de N-éthyloxycarbonyl-4-para-aminophénol par rapport au poids total de la composition.

4. Composition selon l'une quelconque des revendications 1 à 3, caractérisée en ce qu'elle renferme de 0,01 à 5% en poids d'arbutine ou son dérivé par rapport au poids total de la composition.

5. Composition selon la revendication 4, caractérisée en ce qu'elle renferme de 0,1 à 2% en poids d'arbutine ou son dérivé par rapport au poids total de la composition.

6. Composition selon l'une quelconque des revendications 1 à 5, caractérisée en ce qu'elle renferme de 0,001 à 5% en poids d'acide ellagique ou son dérivé par rapport au poids total de la composition.

7. Composition selon la revendication 6, caractérisée en ce qu'elle renferme de 0,005 à 3% en poids d'acide ellagique ou son dérivé par rapport au poids total de la composition.

8. Composition selon l'une quelconque des revendications 1 à 7, caractérisée en ce qu'elle renferme en outre au moins un filtre UV et/ou au moins un agent kératolytique et/ou desquamant.

9. Utilisation de la composition selon l'une quelconque des revendications 1 à 8 dans une préparation cosmétique dépigmentante et/ou blanchissante de la peau humaine, des poils ou des cheveux.

10. Utilisation de la composition selon l'une quelconque des revendications 1 à 8 pour la fabrication d'une préparation dépigmentante et/ou blanchissante de la peau humaine, des poils ou des cheveux.

11. Procédé cosmétique de dépigmentation et/ou de blanchiment de la peau, des poils et/ou des cheveux, consistant à appliquer la composition selon l'une quelconque des revendications 1 à 8 sur la peau humaine, les poils et/ou les cheveux.